# FASCICULE DE BREVET EUROPEEN

(11) **EP 3 361 930 B1**
(45) Date de publication et mention de la délivrance du brevet: **27.01.2021**
(21) Numéro de dépôt: 16794706.8
(22) Date de dépôt: 14.10.2016
(51) Int. Cl.: A61B 3/113, A61B 3/032, G02C 7/02

(54) **MÉTHODE DE DÉTERMINATION D'UN PARAMÈTRE DE COMPORTEMENT VISUEL D'UN INDIVIDU ET DISPOSITIF DE TEST ASSOCIÉ**
VERFAHREN ZUR BESTIMMUNG EINES VISUELLEN VERHALTENSPARAMETERS EINER PERSON UND ZUGEHÖRIGE TESTVORRICHTUNG
METHOD FOR DETERMINING A VISUAL BEHAVIOR PARAMETER OF A PERSON, AND RELATED TESTING DEVICE

(30) Priorité: 15.10.2015 FR 1559811
(43) Date de publication de la demande: 22.08.2018
(73) Titulaire: Essilor International, 94220 Charenton-le-Pont (FR)
(72) Inventeur: ESCALIER, Guilhem, 94220 Charenton-Le-Pont (FR); POULAIN, Isabelle, 94220 Charenton-Le-Pont (FR); DEBIEUVRE, Amandine, 94220 Charenton-Le-Pont (FR); PAILLE, Damien, 94220 Charenton-Le-Pont (FR); TRANVOUEZ-BERNARDIN, Delphine, 94220 Charenton-Le-Pont (FR); BARANTON, Konogan, 94220 Charenton-Le-Pont (FR); ROUSSEAU, Benjamin, 94220 Charenton-Le-Pont (FR)
(74) Mandataire: Jacobacci Coralis Harle
(86) Numéro de dépôt international: PCT/FR2016/052666
(87) Numéro de publication internationale: WO 2017/064442

(56) Documents cités:
- WO-A1-99/22638
- WO-A1-2010/119183
- US-A1- 2006 189 886
- US-B1- 8 950 864

## Description

### DOMAINE TECHNIQUE AUQUEL SE RAPPORTE L'INVENTION

La présente invention concerne de manière générale une méthode de détermination d'un paramètre de comportement visuel d'un individu.

Elle concerne également un dispositif de test pour la mise en œuvre de cette méthode de détermination.

### ARRIERE-PLAN TECHNOLOGIQUE

La personnalisation toujours plus précise des lentilles ophtalmiques d'une monture destinée à équiper un individu pour corriger sa vision nécessite une connaissance accrue du comportement visuel de l'individu dans des conditions de vision naturelles et représentatives de l'utilisation réelle desdites lentilles ophtalmiques.

La détermination de paramètres du comportement visuel de l'individu permet alors d'améliorer la conception optique des lentilles ophtalmiques qui seront montées dans la monture.

En particulier, lors de la conception optique des lentilles ophtalmiques progressives, il est particulièrement important de disposer de données de conception optique qui soient pertinentes pour rendre compte de l'utilisation de ces lentilles en vision de près et de la posture prise par l'individu, notamment en situation de lecture.

Cependant, les mesures effectuées actuellement par l'opticien sur l'individu sont le plus souvent réalisées après les mesures d'ajustement de la monture. Ces mesures sont donc contraintes et liées à une monture donnée, de sorte qu'elles ne sont pas en pratique réutilisables avec une autre monture.

De plus, la monture peut influencer le comportement visuel ou la posture de l'individu de sorte que les mesures sont faites dans des conditions dans lesquelles l'individu n'est pas dans sa posture naturelle.

La détermination de paramètres de comportement visuel de l'individu en conditions naturelles est donc souvent difficile.

Il en résulte que la conception optique des lentilles ophtalmiques à partir de ces paramètres visuo-posturaux n'est pas optimale de sorte que l'individu peut ressentir une gêne lors de l'utilisation de telles lentilles ophtalmiques. Ceci est en particulier vrai pour les lentilles ophtalmiques progressives dont la conception optique doit être réalisée avec soin afin d'assurer une adaptation optimale pour l'individu. Le document de l'art antérieur WO99/22638 divulgue une méthode de détermination d'au moins un paramètre de comportement visuel d'un individu selon le préambule de la revendication 1.

### OBJET DE L'INVENTION

Afin de remédier à l'inconvénient précité de l'état de la technique, la présente invention propose une méthode permettant de déterminer de manière simple et précise le comportement visuel naturel d'un individu.

Plus particulièrement, on propose selon l'invention une méthode de détermination d'au moins un paramètre de comportement visuel d'un individu comprenant les étapes suivantes :
- une étape de sollicitation de l'individu pour qu'il effectue un test visuel au cours duquel il observe au moins une position cible,
- une étape de mesure d'au moins une donnée représentative d'au moins une direction de regard de l'individu au cours dudit test visuel,
- une étape de détermination d'une direction de regard de référence, en fonction de ladite au moins une donnée représentative mesurée,
- une étape de positionnement, par rapport à ladite direction de regard de référence, d'au moins une position cible mesurée qui est déterminée, dans un repère lié à la tête de l'individu, en fonction de ladite donnée représentative de ladite direction de regard de l'individu mesurée au cours du test visuel.

Avantageusement, on réalise, après ladite étape de positionnement, une étape de déduction, en fonction de ladite au moins une position cible mesurée et/ou de la direction de regard de référence, dudit paramètre de comportement visuel de l'individu.

Ainsi, grâce à la méthode selon l'invention, il est possible de tester le comportement visuel de l'individu en conditions naturelles et de déterminer un paramètre de comportement visuel précis de l'individu.

De plus, cette détermination peut être indépendante d'une quelconque monture de sorte que le paramètre peut être réutilisé *a posteriori.*

D'autres caractéristiques non limitatives et avantageuses de la méthode de détermination conforme à l'invention sont les suivantes :
- ladite direction de regard de référence est une direction de regard moyenne de l'individu au cours du test visuel ;
- ladite direction de regard de référence correspond à une direction d'observation d'une cible éloignée quand l'individu est dans une posture naturelle ;
- à l'étape de sollicitation, l'individu observe successivement différentes positions cibles ;
- on détermine lesdites directions de regard de l'individu au cours du test visuel dans un repère lié à la tête de l'individu, puis on détermine les coordonnées desdites positions cibles dans ledit repère lié à la tête de l'individu, et on définit ladite direction de regard de référence comme étant une droite reliant un centre de rotation de l'œil gauche ou droit de l'individu, ou un barycentre desdits centres de rotation, à une position cible dans le repère lié à la tête de l'individu ;
- on détermine lesdites directions de regard de l'individu au cours du test visuel dans un repère lié à la tête de l'individu, on détermine les coordonnées desdites positions cibles dans ledit repère lié à la tête de l'individu, puis on détermine, à partir desdites coordonnées, un barycentre desdites positions cibles dans le repère lié à la tête de l'individu, et on définit ladite direction de regard de référence comme une droite reliant un centre de rotation d'un œil gauche ou droit de l'individu, ou un barycentre desdits centres de rotation, audit barycentre des positions cibles dans le repère lié à la tête de l'individu ;
- ledit repère lié à la tête de l'individu a pour origine l'un des centres de rotation de l'œil droit ou de l'œil gauche de l'individu ou un barycentre desdits centres de rotation ;
- ledit repère lié à la tête de l'individu comprend un premier axe qui est parallèle à une direction de regard primaire de l'individu, un deuxième axe qui est horizontal et perpendiculaire audit premier axe, et un troisième axe qui est perpendiculaire auxdits premier et deuxième axe ;
- la méthode de détermination comporte une étape additionnelle de positionnement, par rapport à ladite direction de regard de référence, de positions cibles théoriques dont les dispositions relatives les unes par rapport aux autres sont identiques aux dispositions relatives desdites positions cibles ;
- lors de ladite étape additionnelle de positionnement, on positionne lesdites positions cibles théoriques de sorte que le barycentre desdites positions cibles théoriques se trouve sur la direction de regard de référence ;
- lors du test visuel, les positions cibles sont disposées sur une surface d'affichage, et, lors de l'étape de positionnement, on détermine une surface d'affichage fictive orientée, par rapport à ladite direction de regard de référence, selon une orientation moyenne de ladite surface d'affichage lors du test visuel et on détermine lesdites positions cibles mesurées comme les intersections desdites directions de regard de l'individu au cours du test visuel et de ladite surface d'affichage fictive ;
- lors de l'étape de déduction, on compare lesdites positions cibles théoriques et lesdites positions cibles mesurées et on en déduit ledit paramètre de comportement visuel de l'individu ;
- lors de l'étape de déduction, on détermine des écarts entre lesdites positions cibles théoriques et lesdites positions cibles mesurées selon une direction privilégiée de ladite surface fictive et on en déduit ledit paramètre de comportement visuel de l'individu ;
- à l'étape de déduction, ledit paramètre de comportement visuel est déterminé en fonction d'un traitement statistique des écarts entre lesdites positions cibles théoriques et lesdites positions cibles mesurées ;
- à l'étape de mesure, on capture, au moyen d'un appareil de capture d'image, au moins une image d'une partie de la tête de l'individu observant chaque position cible, on mémorise ladite au moins une image en association avec les coordonnées, exprimées dans un repère lié à l'appareil de capture d'image, de la position cible observée par l'individu, et on détermine, à partir de ladite au moins une image capturée et des coordonnées associées de la position cible observée, les coordonnées d'un repère lié à la tête de l'individu dans le repère lié audit appareil de capture d'image ou les coordonnées des directions de regard de l'individu dans le repère lié à la tête de l'individu ;
- chaque direction de regard de l'individu est définie comme la droite passant, d'une part, par l'un des centres de rotation de l'œil droit ou de l'œil gauche de l'individu ou le barycentre desdits centres de rotation et, d'autre part, par la position cible observée par l'individu au moment de la mesure ;
- le test visuel effectué par l'individu comporte une tâche visuelle en vision de près destinée à tester le comportement visuel de l'individu en situation de lecture d'un texte ;
- lesdites positions cibles sont alignées selon au moins deux lignes ou deux colonnes sensiblement parallèles et les positions cibles observées successivement, au cours du temps, par l'individu définissent un protocole de suivi visuel destiné à reproduire le déplacement du regard de l'individu pendant la lecture.

L'invention propose également une méthode de conception optique d'une lentille ophtalmique de correction visuelle d'un individu, utilisant le paramètre de comportement visuel déterminé précédemment.

Dans un mode de réalisation préféré, ladite lentille ophtalmique de correction visuelle étant destinée à équiper une monture choisie par l'individu, on réalise, lors de l'étape de déduction de la méthode de détermination du paramètre de comportement visuel, les sous-étapes suivantes :
- on détermine une position relative d'une surface ou d'une ligne liée à ladite monture ou à ladite lentille ophtalmique dans le repère lié à la tête de l'individu,
- on détermine, pour chaque direction de regard de l'individu correspondant à une position cible, l'intersection entre cette direction du regard et ladite surface ou ladite ligne, de manière à établir une cartographie de ces points d'intersection avec ladite surface ou ladite ligne,
- on déduit de cette cartographie un paramètre de conception optique recherché.

L'invention propose enfin un dispositif de test particulièrement adapté à la mise en œuvre de la méthode de détermination précitée.

Selon cette invention, le dispositif de test comporte :
- un afficheur actif adapté à afficher au moins une cible visuellement prépondérante en une pluralité de positions cibles alignées selon au moins deux lignes ou colonnes sensiblement parallèles,
- une unité de commande de l'afficheur, programmée pour que les positions cibles définissent, au cours du temps, un protocole de suivi visuel de manière à reproduire le déplacement du regard de l'individu pendant la lecture, et
- un appareil de capture d'image piloté par ladite unité de commande de manière synchrone avec ledit afficheur pour déclencher des captures d'images de la tête de l'individu observant ladite cible affichée par l'afficheur, chaque image capturée correspondant à une position cible prédéterminée.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique d'un individu tenant dans ses mains un dispositif de test conforme à l'invention ;
- la figure 2 est une vue de face du dispositif de test de la figure 1 sur lequel est affichée une cible visuelle se déplaçant selon un protocole de suivi visuel ;
- la figure 3 est une vue schématique de la tête de l'individu et de différents plans associé à cette tête ;
- la figure 4 représente un repère lié à la tête de l'individu ;
- la figure 5 représente un afficheur du dispositif de test de la figure 1 avec une cible affichée et un repère lié à la tête de l'individu regardant la cible dans une position finale du protocole ;
- les figures 6 et 7 représentent des exemples de positions mesurées de la cible dans le référentiel lié à la tête de l'individu au cours du protocole de lecture ;
- la figure 8 est un schéma de principe montrant une direction de regard de référence dans un référentiel lié à la tête de l'individu et une surface d'affichage fictive des positions cibles théoriques ;
- la figure 9 représente dans le référentiel lié à la tête de l'individu les positions cibles théoriques sur la surface d'affichage et les positions cibles mesurées dans ce référentiel ;
- la figure 10 est un graphe illustrant le calcul de l'écart entre les positions cibles théoriques et les positions cibles mesurées ;
- la figure 11 est une courbe représentative des écarts entre les positions cibles théoriques et les positions cibles mesurées en fonction des positions cibles théoriques ;
- les figures 12 et 13 sont des courbes illustrant le calcul de paramètres du comportement visuel de l'individu lorsqu'il regarde la cible du protocole de la figure 2.

En préambule, on notera que les éléments identiques ou similaires des différents modes de réalisation représentés sur les différentes figures seront référencés par les mêmes signes de référence et ne seront pas décrits à chaque fois.

On notera également que dans l'exposé qui va suivre, les termes « haut » ou « supérieur ») et « bas » (ou « inférieur ») seront utilisés relativement à l'individu utilisant le dispositif de test, le haut désignant le côté tourné vers la tête de l'individu et le bas désignant le côté tourné vers les pieds de l'individu.

De même, le terme « avant » désignera le côté tourné vers l'individu, le terme « arrière » désignant le côté opposé au côté avant.

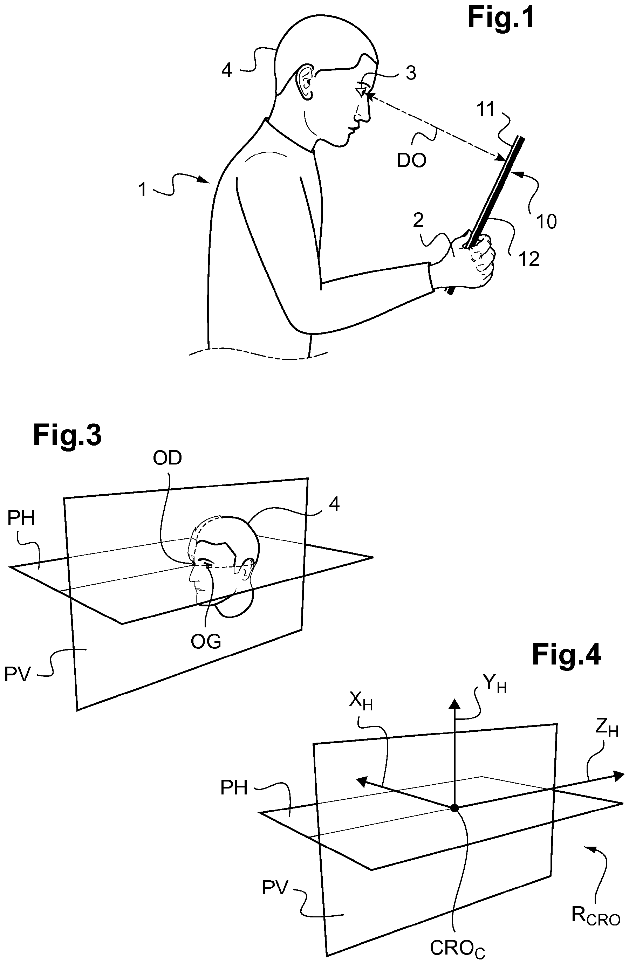

Sur la figure 1, on a représenté un individu 1 dont on souhaite tester le comportement visuel.

À cet effet, l'individu 1 tient dans ses mains 2 un dispositif de test 10 conforme à l'invention destiné à déterminer ce comportement visuel dans des conditions données.

Plus particulièrement ici, on souhaite utiliser le dispositif de test 10 pour analyser de manière générale la vision de près de l'individu 1, et en particulier le comportement visuel qu'il adopte lorsqu'il est en situation de lecture.

On considérera que la vision de près correspond à une distance d'observation DO (voir figure 1) entre l'œil 3 de l'individu 1 et le dispositif de test 10 inférieure à 70 centimètres (cm).

Dans d'autres modes de réalisation, la vision intermédiaire (DO comprise entre 40 cm et 4 mètres) ou la vision de loin (DO supérieure à 4 m) peuvent être testées grâce au dispositif de test selon l'invention.

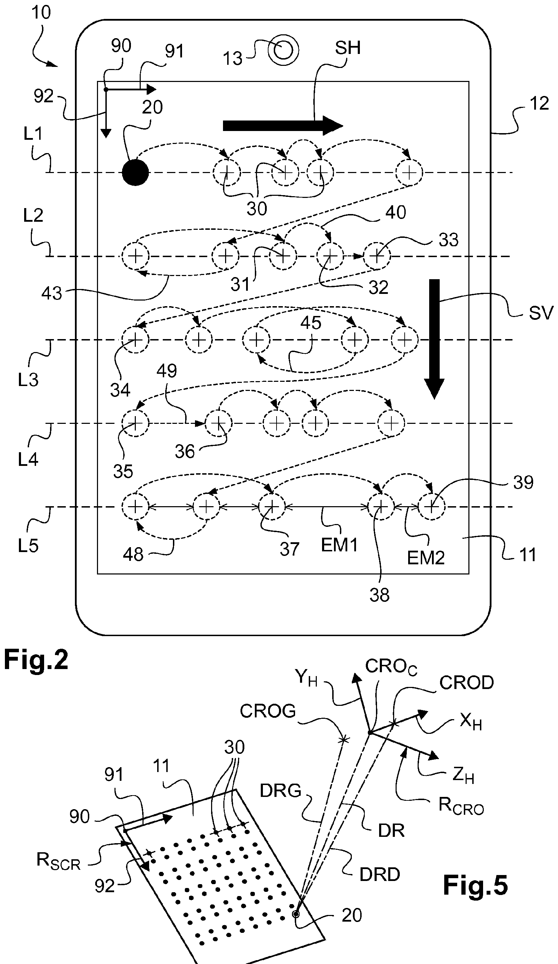

Le dispositif de test 10 comporte (voir figures 1 et 2) :
- un afficheur 11 actif qui affiche une cible 20 visuellement prépondérante en une pluralité de positions cibles 30 alignées selon au moins deux lignes ou colonnes sensiblement parallèles, et
- une unité de commande (non représentée) de l'afficheur 11, programmée pour que les positions cibles 30 définissent, au cours du temps, un protocole de suivi visuel de manière à reproduire le déplacement du regard de l'individu pendant la lecture.

L'afficheur 11 du dispositif de test peut afficher, à chaque instant du test visuel, une seule et unique cible ou bien plusieurs cibles simultanément. Dans les deux cas, la cible visuellement prépondérante est celle qui est adaptée à accrocher le regard de l'individu et que l'individu va suivre au cours du test visuel.

Lorsque plusieurs cibles sont affichées par l'afficheur 11, la cible visuellement prépondérante peut être, par exemple, une cible plus lumineuse ou plus contrastée, de couleur ou de forme (rond, carré, étoile, ...) différente, ou de taille plus petite ou plus grande que les autres, ou bien encore une cible qui clignote alors que les autres ne clignotent pas. Les différentes cibles affichées par l'afficheur peuvent également comprendre un ensemble d'indicateurs ou bien former une grille de points gris.

Dans les modes de réalisation où l'afficheur 11 n'affiche qu'une seule cible 20 (cas de la figure 2), celle-ci peut prendre une pluralité de positions cibles 30 sur l'afficheur 11. Ces positions cibles 30 sont « variables » en ce sens que la cible 20 se déplace séquentiellement d'une position cible 30 à une autre au cours du test visuel. On notera néanmoins que la séquence des positions cibles 30 prises successivement par la cible 20 dans ces modes de réalisation peut comprendre deux positions cibles 30 identiques. En d'autres termes, il est possible qu'au cours du test visuel la cible 20 repasse par une position cible 30 déjà prise précédemment.

Dans les modes de réalisation où l'afficheur affiche plusieurs cibles dont l'une est visuellement prépondérante, les positions d'affichage des cibles peuvent être variables au cours du temps, mais en tout état de cause, la cible visuellement prépondérante est celle qui se déplace selon une séquence de positions cibles de manière à imposer à l'individu 1 une succession de directions de regard particulières.

Dans la présente description, on entendra par « *protocole de suivi visuel* » la séquence d'affichage de la cible 20 visuellement prépondérante au cours du test visuel réalisé par l'individu 1.

En d'autres termes, ce protocole de suivi visuel correspond à la succession, dans le temps, des positions cibles 30 prises par la cible 20 visuellement prépondérante. Grâce à celle-ci, on impose un protocole à l'individu 1 qui regarde successivement dans une pluralité de directions particulières désirées qui sont chacune associées à une position cible 30 particulière prise par la cible 20. De cette façon, si les positions cibles 30 de cette cible 20 sont connues, il est alors possible, dans certaines conditions, de remonter à l'information concernant la direction de regard de l'individu 1 lors du test visuel.

Dans la suite de la description, on entendra par « direction de regard » de l'individu 1 associée à une position cible 30 de la cible 20, la direction de la droite passant par :
- l'un des centres de rotation de l'œil droit ou de l'œil gauche de l'individu 1, ou un barycentre de ces centres de rotation ; et
- ladite position cible 30 lorsque l'individu 1 observe la cible 20 prenant cette position cible 30.

Comme illustré sur la figure 2, le dispositif de test 10 se présente ici sous la forme d'une tablette numérique. Cette tablette numérique comprend un écran qui constitue l'afficheur 11 du dispositif de test 10. Elle comprend également un boîtier 12 entourant l'écran. L'unité de commande du dispositif de test 10 correspond, quant à elle, au contrôleur d'affichage de l'écran 11 de la tablette qui est logé à l'intérieur du boîtier 12.

Le dispositif de test 10 comporte également un appareil de capture d'image 13 qui est piloté par l'unité de commande de manière synchrone avec l'afficheur 11 pour déclencher des captures d'images de la tête 4 de l'individu 1 observant la cible 20 affichée par l'afficheur 11, chaque image capturée correspondant à une position cible 30 prédéterminée.

De préférence, on utilise ici la caméra frontale 13 intégrée à la tablette 10 comme appareil de capture d'image du dispositif de test. Cette caméra frontale 13 présente l'avantage de toujours faire face et de viser l'individu 1 lors du test visuel effectué par l'individu 1.

Dans d'autres modes de réalisation, on peut prévoir d'utiliser un appareil de capture d'image séparé et distinct de l'afficheur.

La cible 20 comprend ici un disque lumineux qui est affiché sur l'écran de la tablette, la taille de la cible étant suffisante pour qu'elle soit visible par l'individu 1 dans les conditions du test visuel. Ici, en conditions de lecture et en vision de près (DO < 70 cm), la cible 20 a une taille caractéristique (par ex. diamètre) supérieure à 5 millimètres.

De manière avantageuse, la taille caractéristique de la cible 20 est déterminée de telle sorte qu'elle puisse être vue avec une acuité supérieure à 0.1 dixième à 70 cm.

En variante, la cible peut comprendre un motif géométrique, régulier ou non. Il s'agit de préférence d'un motif quelconque, à l'exclusion d'un signe utilisé par un système d'écriture quelconque compréhensible par l'individu. En particulier, la cible visuellement prépondérante est dénuée de signification pour l'individu. Par exemple, la cible n'est pas un mot intelligible pour l'individu.

On va maintenant décrire, en référence à la figure 2, le protocole de suivi visuel qui est mis en œuvre par le dispositif de test 10 et qui est destiné ici à simuler la lecture d'un texte par l'individu 1.

De manière avantageuse, l'affichage de la cible selon le protocole de suivi visuel mis en œuvre par le dispositif de test 10 constitue un stimulus visuel pour l'individu 1, destiné à lui faire bouger les yeux 3 par suivi de cette cible 20 selon le même schéma que celui que l'individu 1 adopterait s'il lisait réellement un texte.

En d'autres termes, l'affichage de la cible 20 visuellement prépondérante sur l'afficheur 11 est commandé de telle sorte que, lorsque l'individu 1 suit du regard la cible 20 d'une position cible 30 à une autre, la direction du regard de l'individu 1 présente des directions de regard successives tout à fait similaires aux directions de regard qu'aurait cet individu 1 en lisant un texte.

La séquence des positions cibles 30 prises successivement par la cible 20 visuellement prépondérante est de préférence prédéterminée en fonction d'un texte de référence, et/ou d'un modèle de lecture, correspondant aux caractéristiques et/ou aux préférences de lecture/écriture de l'individu.

Par exemple, la séquence peut être prédéterminée préalablement avec un autre dispositif, au cours d'une opération de calibration lors de laquelle on demande à l'individu de choisir un texte de référence parmi une pluralité de textes réels disponibles et de le lire à voix haute. La vitesse de lecture peut alors servir de paramètre pour la détermination des positions d'affichage de la cible.

La séquence peut également être prédéterminée en fonction de l'âge de l'individu ou en fonction d'un niveau de lecture déclaré par l'individu, à la suite d'un questionnaire rempli par l'individu.

On peut également envisager de faire un entraînement avec une vitesse moyenne, demander à l'individu si cette vitesse moyenne était trop ou pas assez rapide et ajuster la vitesse en fonction de sa réponse.

On observera tout d'abord que la lecture d'un texte par un individu se fait naturellement selon un schéma de lecture comportant trois opérations distinctes : fixations, saccades et rétro-saccades.

Lors des fixations, l'individu déchiffre le mot qu'il est en train de lire, c'est-à-dire le mot sur lequel le regard de l'individu est fixé.

Lors des saccades, correspondant aux phases de déplacement, c'est-à-dire aux passages de la lecture d'un mot au mot suivant, les yeux de l'individu bougent rapidement pour passer d'une fixation à une autre.

Ces saccades sont liées à l'empan visuel, c'est-à-dire au nombre de caractères (lettres, symboles, idéogrammes, etc...) qui sont déchiffrables pour une fixation donnée. Elles permettent au lecteur de déchiffrer tous les caractères d'un texte.

Les saccades se font généralement dans le sens de la lecture du texte. Néanmoins, les yeux effectuent également des « rétro-saccades » très rapides dans le sens opposé au sens de lecture pour passer d'une fixation à une autre. Ce mouvement est induit par une erreur des muscles oculomoteurs ou par une mauvaise lecture et compréhension du texte.

Un des avantages du dispositif de test 10 est de proposer des protocoles de suivi visuel qui se rapprochent le plus possible des schémas de lecture de l'individu.

Le dispositif de test 10 permet donc de simuler simplement la lecture d'un texte et de placer l'individu dans une situation où il adoptera une posture naturelle proche de celle qu'il adopterait pour une lecture en vision de près.

Une détermination du comportement visuel de l'individu dans ces conditions est donc rendue plus précise et la conception optique d'une lentille ophtalmique destinée à l'individu peut être améliorée afin que le design de la lentille ophtalmique réponde aux besoins visuels de l'individu.

De préférence, les positions cibles 30 de la cible 20 sont alignées selon au moins deux lignes sensiblement parallèles. Plus précisément, sur l'exemple de réalisation montré sur les figures, l'unité de commande de l'afficheur 11 est programmée pour que les positions cibles 30 successives de la cible 20 soient alignées sur cinq lignes L1, L2, L3, L4, L5 (voir figure 2).

Alternativement, les positions cibles de la cible peuvent être alignées selon au moins deux colonnes.

De manière générale, les positions cibles 30 de la cible 20 peuvent définir des lignes parallèles de direction quelconque, notamment sensiblement horizontale ou verticale pour l'individu 1.

De préférence encore, chaque ligne, ou alternativement chaque colonne, comprend au moins trois positions alignées de ladite cible (cas des positions 35, 36, 37, 38, 39 pour la ligne L5 de la figure 2).

Afin que le protocole de suivi visuel soit le plus représentatif d'une lecture par le porteur, il est avantageusement prévu que le protocole de suivi visuel décrive un trajet de lecture qui soit conforme à celui défini par un système d'écriture donné, de manière à reproduire le déplacement du regard de l'individu pendant la lecture conformément au système d'écriture.

Le trajet de lecture peut être ici défini comme le chemin, au niveau de l'afficheur 11, balayé par la direction de regard de l'individu 1 lorsqu'il regarde la séquence des positions cibles 30 prises par la cible 20 visuellement prépondérante.

Le schéma de lecture adopté par un individu est lié non seulement à la nature ou aux propriétés spécifiques du texte, mais aussi aux spécificités de chaque écriture.

On notera d'ailleurs que les différentes écritures peuvent être classifiées de façon fonctionnelle (écriture alphabétique, syllabique ou logographique) et directionnelle (sens horizontal et vertical d'écriture et/ou de lecture).

On prévoit donc dans le dispositif de test que l'unité de commande mémorise un sens de parcours vertical SV et horizontal SH (voir figure 2) privilégié du protocole de suivi visuel.

Ce sens de parcours vertical et horizontal privilégié est préalablement déterminé en fonction des caractéristiques de l'individu, et en particulier sa capacité à lire un texte selon un système d'écriture donné.

Par exemple, lorsque le dispositif de test est utilisé par un français qui lit de droite à gauche et de haut en bas, le sens de parcours horizontal mémorisé par l'unité de commande est un sens de parcours allant de la gauche de l'écran 11 à la droite de l'écran 11, et le sens de parcours vertical mémorisé par l'unité de commande est un sens de parcours allant du haut de l'écran 11 au bas de l'écran 11.

Aussi, dans un mode de réalisation préféré, les lignes L1, L2, L3, L4, L5 sensiblement parallèles le long desquelles sont alignées les positions cibles 30 de la cible 20 s'étendent sensiblement horizontalement, le sens de parcours du protocole de suivi visuel étant identique pour toutes les lignes prises successivement de la plus haute à la plus basse, de gauche à droite (ou de droite à gauche pour une écriture de droite à gauche comme l'arabe ou l'hébreu).

De la même manière, lorsque le dispositif de test est utilisé par un mongolien, qui lit de haut en bas et de droite à gauche, le sens de parcours vertical mémorisé par l'unité de commande est un sens de parcours allant du haut de l'écran au bas de l'écran, et le sens de parcours horizontal mémorisé par l'unité de commande est un sens de parcours allant de la droite de l'écran à la gauche de l'écran.

Aussi, dans un mode de réalisation adapté à ce système d'écriture, les lignes sensiblement parallèles le long desquelles sont alignées les positions prédéterminées de la cible s'étendent sensiblement verticalement, le sens de parcours du protocole de suivi visuel étant identique, de haut en bas ou de bas en haut, pour toutes les lignes prises successivement de droite à gauche.

De manière avantageuse, l'unité de commande du dispositif de test 10 est programmée pour permettre la sélection du protocole de suivi visuel parmi une pluralité de protocoles de suivi visuel enregistrée dans une base de données locale ou distante, dans laquelle un sens de parcours est enregistré en association avec le protocole de suivi visuel auquel il correspond.

Ainsi, l'individu en fonction de ses caractéristiques propres de lecture et/ou d'écriture peut choisir le protocole visuel qui lui correspond, de sorte qu'il est dans des conditions naturelles proches de la lecture lors de la réalisation du test visuel. On s'assure alors de mettre en place ses mécanismes et ses stratégies de lecture afin de récupérer la posture la plus représentative de l'utilisation de sa vision de près.

Afin de reproduire le schéma de lecture tel que décrit plus haut, avec fixations, saccades et rétro-saccades, il est prévu que l'unité de commande de l'afficheur 11 affiche la cible 20 selon un protocole de suivi visuel préférentiel.

Aussi, il est prévu que l'unité de commande impose, dans chaque position cible 30 du protocole de suivi visuel, que la cible 20 s'affiche pendant une durée prédéterminée. On entend par là que la cible 20 est maintenue affichée fixement sur l'écran de manière à ce qu'on force l'individu 1 à fixer son regard sur la cible 20, ce qui correspond à une fixation sur la position cible 30 dans le trajet de lecture de l'individu 1.

De manière avantageuse, la cible 20 est fixe pendant la durée prédéterminée, c'est-à-dire que la position cible 30 de la cible 20 pendant cette durée prédéterminée ne change pas, avant le passage à la position cible suivante du trajet de lecture.

De préférence, cette durée prédéterminée est comprise entre 50 millisecondes et 1 seconde, ce qui correspond typiquement à des temps de fixation standard.

La durée prédéterminée peut également varier au cours du trajet de lecture, ceci rendant compte du fait que la fixation du regard de l'individu 1 sur un mot lors d'une lecture réelle peut dépendre du mot (taille, longueur) et du niveau de compréhension de ce mot (mot mal connu ou inconnu, mot ou caractère peu déchiffrable, mot mal orthographié, etc...).

De manière avantageuse également, il est prévu que l'unité de commande impose un délai prédéterminé entre les affichages de la cible 20 dans deux positions cibles successives (voir par exemple les positions cibles 31, 32 sur la figure 2) du protocole de suivi visuel.

De cette façon, on peut simuler grâce au dispositif de test 10 les saccades ou rétro-saccades existant lors du trajet de lecture de l'individu 1. Comme précédemment, on peut prévoir que l'unité de commande fasse varier le délai prédéterminé au cours du protocole de suivi visuel.

Ceci permet de rendre compte que la vitesse de lecture de l'individu 1 peut varier au cours de la lecture d'un texte.

Ceci permet également d'envisager les cas où la direction de regard de l'individu 1 passe d'une ligne à une autre, comme c'est le cas par exemple de la position cible 33 à la position cible 34 de la figure 2, le retour à la ligne nécessitant plus de temps dans la mesure où la variation de direction de regard de l'individu 1 est plus importante.

Il est alors possible de prévoir deux cas pour la cible lors du délai prédéterminé.

Dans un mode de réalisation, on peut prévoir que la cible est invisible pendant le délai prédéterminé. Ceci correspond au cas des positions cibles 31 et 32 de la figure 2 où la cible 20 « saute » (le saut étant représenté par la flèche pointillée 40) de la position 31 à la position suivante 32. Ce mode de réalisation permet de rendre compte du regard de l'individu qui saute de mot en mot lors de la lecture d'un texte.

Dans un mode de réalisation alternatif, on peut prévoir que la cible est visible pendant le délai prédéterminé et se déplace entre les deux positions cibles successives correspondantes du protocole de suivi visuel, de l'une à l'autre. Ceci correspond au cas des positions cibles 35 et 36 où la cible se déplace (le déplacement étant représenté par la flèche en pointillés 49), tout en restant visible.

De manière avantageuse, le dispositif de test 10 de l'invention est tel que l'unité de commande impose que deux positions cibles 37, 38, 39 successives du protocole de suivi visuel sont séparées d'une distance EM1, EM2 inférieure à 10 centimètres. De cette façon, lors du test visuel, on ne sollicite pas l'individu 1 de telle sorte que la variation de sa direction de regard ne soit pas trop importante, ce qui en condition de lecture est généralement le cas.

Préférentiellement, il est par ailleurs prévu que l'unité de commande impose que la distance EM1, EM2 séparant deux positions cibles 37, 38, 39 successives du protocole de suivi visuel varie le long du protocole de suivi visuel. Ceci permet d'adapter l'écart entre les cibles 20 affichées en fonction de l'empan moyen des mots pour un système d'écriture donné.

Dans un autre mode de réalisation, l'unité de commande est programmée pour que l'affichage de la cible 20 dans deux positions cibles successives du protocole de suivi visuel suive le sens de parcours privilégié, horizontal et/ou vertical, au moins six fois sur dix. Ceci est illustré sur la figure 2 sur laquelle on a représenté dans le protocole de suivi visuel, des sens de parcours, représentés par les flèches pointillées 43, 45, 48, qui vont non pas de gauche à droite comme le sens de parcours horizontal SH privilégié, mais de droite à gauche.

Il est ainsi possible grâce à cela de simuler les mouvements de rétro-saccades précédemment décrits lors de la lecture d'un texte par l'individu 1. En effet, ici quatre fois sur dix, le mouvement des yeux 3 de l'individu 1 suivant la cible 20 du regard entre deux positions cibles 30 successives se fait dans le sens opposé au sens privilégié de parcours.

Comme pour les mouvements de saccades détaillés ci-dessus, la cible 20 peut passer d'une position cible à la position cible suivante, dans un sens de parcours opposé au sens de parcours privilégié, soit en sautant d'une position à l'autre (cible invisible), soit en se déplaçant de l'une à l'autre (cible visible).

On va maintenant décrire, en référence aux figures 3 à 13, une méthode de détermination d'au moins un paramètre de comportement visuel de l'individu 1, cette méthode utilisant le dispositif de test décrit ci-dessus qui est particulièrement adapté à la mise en œuvre de cette méthode.

Selon l'invention, la méthode de détermination comprend les étapes suivantes :
- une étape de sollicitation de l'individu pour qu'il effectue un test visuel au cours duquel il observe au moins une position cible,
- une étape de mesure d'une donnée représentative d'au moins une direction de regard de l'individu au cours dudit test visuel,
- une étape de détermination d'une direction de regard de référence, en fonction desdites données représentatives mesurées,
- une étape de positionnement, par rapport à ladite direction de regard de référence, d'au moins une position cible mesurée qui est déterminée en fonction de ladite donnée représentative de ladite direction de regard de l'individu mesurée au cours du test visuel.

Avantageusement, on réalise, après l'étape de positionnement, une étape de déduction, en fonction de ladite au moins une position cible mesurée, du paramètre de comportement visuel de l'individu.

En pratique, la tablette 10, ou un ordinateur local ou distant, est programmée pour accomplir les étapes ci-dessus et détaillées ci-après.

De préférence, à l'étape de sollicitation de la méthode de détermination, l'individu 1 observe successivement différentes positions cibles 30.

L'individu 1 est donc sollicité pour observer l'écran 11 de la tablette 10 qui affiche la cible 20 visuellement prépondérante selon une séquence prédéterminée de positions cibles 30 du protocole de suivi visuel choisi tel que décrit plus haut en référence à la figure 2.

Selon une première variante de réalisation, la méthode de détermination comprend des étapes intermédiaires suivantes :
- on détermine lesdites directions de regard de l'individu au cours du test visuel dans un repère lié à la tête de l'individu,
- on détermine les coordonnées desdites positions cibles dans ledit repère lié à la tête de l'individu, et
- on détermine, à partir desdites coordonnées, un barycentre desdites positions cibles dans le repère lié à la tête de l'individu, et
- on définit ladite direction de regard de référence comme une droite reliant un centre de rotation d'un œil gauche ou droit de l'individu, ou un barycentre desdits centres de rotation, audit barycentre des positions cibles dans le repère lié à la tête de l'individu.

Comme référentiel lié à la tête 4 de l'individu 1, on peut par exemple choisir un référentiel dit « *référentiel de regard primaire »* ou « *repère CRO* », dans lequel la tête 4 de l'individu 1 présente une position et une orientation fixe et auquel on associe un repère, de préférence orthonormé, ayant une origine et trois axes non liés.

Les figures 3 et 4 illustrent comment est construit ce repère CRO.

En particulier, on a représenté sur la figure 3 un plan vertical PV correspondant à un plan sagittal de la tête 4 de l'individu 1 qui est le plan vertical passant par une médiatrice des deux yeux droit et gauche OD, OG de l'individu 1.

Cette médiatrice des yeux OD, OG est un axe qui passe au milieu d'un segment qui est défini par le centre de rotation de l'œil droit OD (ci-après référencé CROD) et le centre de rotation de l'œil gauche OG (ci-après référencé CROG) et qui est parallèle au plan de Francfort de la tête 4 de l'individu 1.

Le plan de Francfort de la tête de l'individu est défini comme le plan passant par les points orbitaires inférieurs de l'individu 1 et le porion de l'individu 1, le porion étant le point du crâne le plus élevé du conduit auditif, qui correspond au tragion de l'oreille. Pour la détermination du plan de Francfort, on considère que l'individu est dans une position orthostatique, dans laquelle il réalise un minimum d'efforts. Cette position correspond à une posture naturelle, ci-après désignée *« posture de regard primaire ».*

Dans cette position naturelle, la direction de regard de l'individu est alors la direction de regard primaire, c'est-à-dire qu'il regarde droit devant lui. Le plan de Francfort est alors généralement horizontal.

On définit par ailleurs (voir figure 3) un plan PH qui contient les centres de rotation CROD, CROG des yeux OD, OG de l'individu 1.

Dans l'exemple particulier décrit ici, ce plan PH est parallèle au plan de Francfort de la tête 4 de l'individu 1 et, est donc horizontal.

À partir de la posture de regard primaire de l'individu 1, c'est-à-dire de la connaissance de l'orientation du plan de Francfort, et des centres de rotation CROD, CROG des yeux OD, OG de l'individu 1, il est possible de construire le repère CRO lié à la tête 4 de l'individu 1, ci-après référencé R_{CRO}, en choisissant :
- une origine qui est l'un des centres de rotation CROD, CROG de l'œil droit OD ou de l'œil gauche OG de l'individu 1 ou un barycentre de ces centres de rotation CROD, CROG ;
- un premier axe qui est parallèle à une direction de regard primaire de l'individu 1 ;
- un deuxième axe qui est horizontal et perpendiculaire au premier axe, et
- un troisième axe qui est perpendiculaire au premier axe et au deuxième axe.

Dans les exemples de réalisation décrits, l'origine du repère R_{CRO} est choisie comme étant le point situé au milieu du segment joignant le centre de rotation CROD de l'œil droit OD et le centre de rotation CROG de l'œil gauche OG de l'individu 1. En d'autres termes, ce point d'origine, désigné ci-après *« CRO cyclope* » et référencé CRO_{c} correspond à l'isobarycentre des centres de rotation CROD, CROG des yeux OD, OG de l'individu 1.

Les trois axes X_{H}, Y_{H}, Z_{H}, du repère R_{CRO} sont également représentés sur la figure 4.

L'axe X_{H} (deuxième axe) passe par le CRO cyclope CRO_{C} et est ici orienté du centre de rotation gauche CROG vers le centre de rotation droit CROD. L'axe X_{H} est ici horizontal car contenu dans le plan horizontal PH parallèle au plan de Francfort. Une orientation opposée est également possible.

L'axe Z_{H} (premier axe) est parallèle à la direction de regard primaire lorsque l'individu 1 est dans une position naturelle, c'est-à-dire en posture de regard primaire. Dans le cas particulier décrit ici, l'axe Z_{H} est situé dans le plan vertical PV de la tête 4 de l'individu 1 et est parallèle au plan de Francfort. Dans d'autres cas où la tête de l'individu présente un angle de lacet, cet axe Z_{H} peut ne pas être situé dans le plan vertical. L'axe Z_{H} s'étend ici dans une direction s'éloignant de la tête 4 de l'individu 1 (vers l'arrière).

L'axe Y_{H} (troisième axe) s'étend, quant à lui, dans le plan sagittal vertical PV de la tête 4 de l'individu 1 et est perpendiculaire au plan de Francfort. L'axe Y_{H} est donc bien perpendiculaire à l'axe X_{H} et à l'axe Z_{H}. Il est ici orienté vers le haut, de sorte que le repère R_{CRO} est direct.

On notera que le repère R_{CRO} est lié à la tête 4 de l'individu 1 et que donc ce repère R_{CRO} bouge avec la tête 4 de l'individu 1, la position et l'orientation de ce repère R_{CRO} changeant par rapport à un repère absolu ou de référence (par exemple un repère lié à la pièce dans laquelle l'individu effectue le test visuel) qui ne serait pas lié à la tête 4 de l'individu 1 en fonction des mouvements de la tête 4 de l'individu 1.

On notera que la détermination des positions des centres de rotation CROD, CROG peut être réalisée selon le principe connu en soi et exposé par exemple dans le document FR 2914173, dont un équivalent en anglais est le document US 2010/0128220.

Lors de cette détermination des centres de rotation CROD, CROG, l'individu 1 porte, sur sa tête 4, solidaire de la tête 4, un système de repérage (référentiel métrologique) ou « *clip »* qui comprend des éléments de repérage (marqueurs) détectables lors d'une capture d'image de la tête 4 de l'individu 1.

En résumé, on capture au moins deux images de la tête 4 de l'individu 1 au moyen d'un appareil de capture d'images :
- une première image lorsque l'individu regarde l'appareil de capture d'images en se positionnant de face, en regardant au loin droit devant (posture de regard primaire), et
- une deuxième image lorsque l'individu regarde l'appareil de capture d'images en se positionnant de trois-quart.

À partir d'un traitement des deux images capturées (voir document FR 2914173), on déduit les positions des centres de rotation CROD, CROG dans un référentiel lié au système de repérage.

Il est alors possible de déterminer le centre de rotation « cyclope », qui est l'isobarycentre des deux centres de rotation CROD, CROG précédemment déterminés.

Pour la détermination de la posture de regard primaire, on réutilise les positions des centres de rotation CROD, CROG ainsi que la première image capturée de face. On peut également prévoir de compenser l'inclinaison de la tablette 10 lors de cette dernière détermination.

On a représenté sur la figure 5 la direction de regard DR joignant le CRO cyclope à la cible 20 positionnée ici sur la dernière position cible du protocole de suivi visuel ainsi que le repère R_{CRO} lié à la tête 4 de l'individu 1 avec ses trois axes principaux X_{H}, Y_{H}, Z_{H}.

On a également représenté sur cette figure 5 les directions de regard référencées respectivement DRD et DRG correspondant aux directions de regard pour l'œil droit OD et l'œil gauche OG de l'individu 1.

Une fois que l'on a choisit le repère lié à la tête 4 de l'individu 1, ici le repère R_{CRO}, on peut, pour chaque position cible 30 de la cible 20 observée sur l'écran 11 de la tablette 10, déterminer les coordonnées de ces positions cibles dans ce repère R_{CRO}.

À cet effet, lors de l'étape de mesure de la méthode de détermination :
- on capture, au moyen de la caméra frontale 13 du dispositif de test 10 tournée vers la tête 4 de l'individu 1, des images d'une partie de la tête 4 de l'individu 1 observant chaque position cible 30, chaque position cible 30 pouvant être prédéterminée dans un repère lié à la caméra frontale 13,
- on mémorise ces images en association avec les coordonnées, exprimées dans ce repère lié à la caméra frontale 13, de la position cible 30 observée par l'individu 1, et
- on détermine, à partir des images capturées et des coordonnées associées de la position cible 30 observée, les coordonnées du repère R_{CRO} lié à la tête 4 de l'individu 1 dans le repère lié à l'appareil de capture d'image 13 ou les coordonnées des directions de regard DR de l'individu 1 dans le repère R_{CRO} lié à la tête 4 de l'individu 1.

Un repère lié à la caméra frontale 13 peut être par exemple le repère R_{SCR} de l'écran 11 (voir figure 5 par exemple) qui a pour origine le coin supérieur gauche 90 de l'écran 11 et pour axes les deux axes 91, 92 perpendiculaires entre eux et dirigés selon les colonnes et les lignes de l'écran 11.

Avantageusement, la caméra frontale 13 déclenche une capture d'image de la tête 4 de l'individu 1 avec un décalage de capture par rapport au moment où la cible 20 s'affiche aux positions cibles 30 prédéterminées du protocole de suivi visuel sur l'écran 11. Ce décalage peut être nul, ou bien de préférence faible, par exemple inférieur à 200 millisecondes. Ceci permet de prendre en compte le temps de réaction et de déplacement des yeux 3 de l'individu 1 lors d'un changement de position 30 de la cible 20 sur l'écran 11.

Selon une variante, la caméra frontale peut également réaliser une séquence vidéo en continu, par exemple à une cadence de vingt images par seconde, et extraire de la séquence vidéo la meilleure image donnant la meilleure information sur le comportement visuel de l'individu lors de l'affichage de la cible à la position cible correspondante.

Chaque image capturée par la caméra frontale 13 de la tablette 10 correspond ainsi à une position cible 30 prédéterminée de la cible 20 visuellement prépondérante, dont la position 30 dans le repère R_{SCR} lié à l'appareil de capture d'image 13 est parfaitement connue.

Pour déterminer les coordonnées du repère R_{CRO} lié à la tête 4 de l'individu 1 dans le repère lié à l'appareil de capture d'image 13 ou les coordonnées des directions de regard DR de l'individu 1 dans le repère R_{CRO} lié à la tête 4 de l'individu 1, il est prévu des moyens de traitement d'images de la tablette 10, constitués par exemple par le processeur de la tablette 10, qui détecte dans les images capturées de la tête 4 de l'individu 1 les marqueurs du clip porté par l'individu 1 sur sa tête 4.

On détermine alors pour chaque image capturée, c'est-à-dire pour chaque position cible 30 de la cible 20 du protocole de suivi visuel, la position et l'orientation du clip dans le repère R_{SCR} lié à la caméra frontale 13 par exemple en utilisant le procédé décrit dans le document US 2010/0128220.

Les positions des centres de rotation CROD, CROG des yeux de l'individu 1 par rapport au clip étant connues, la position (coordonnées spatiales) et l'orientation (coordonnées angulaires) du repère R_{CRO} lié à la tête 4 de l'individu 1 sont également connues par rapport au clip.

Ceci est d'ailleurs illustré sur la figure 5 où l'on a représenté le repère R_{CRO} avec son origine au centre de rotation cyclope CRO_{c} (isobarycentre des centres de rotation CROD, CROG) et ses axes X_{H}, Y_{H}, Z_{H}.

Ainsi, par un changement de repère, il est possible de déterminer, pour chaque position cible 30 de la cible 20 du protocole de suivi visuel, la position et l'orientation du repère R_{CRO} lié à la tête 4 de l'individu 1 dans le repère R_{SCR} lié à la caméra frontale 13 de la tablette 10.

On peut également déterminer, pour chaque position cible 30 de la cible 20 du protocole de suivi visuel, les directions de regard DR de l'individu 1 dans le référentiel R_{CRO} lié à la tête 4 de l'individu 1, ces directions de regard DR joignant ici le centre de rotation cyclope CRO_{C}, origine du repère R_{CRO} lié à la tête 4 de l'individu 1, à la cible 20.

On peut enfin réexprimer, à partir des positions et orientations de la tête 4 ou des directions de regard DR de l'individu 1, les positions cibles 30 de la cible 20 dans le repère R_{CRO} lié à la tête 4 de l'individu 1.

Ces positions cibles 30 dans le repère R_{CRO} lié à la tête 4 de l'individu 1 sont des données représentatives des directions de regard DR mesurées de l'individu 1 lors du protocole de suivi visuel.

Selon l'invention, on détermine, après l'étape de mesure, une direction de regard de référence en fonction de ces données représentatives.

Dans certains modes de réalisation, la direction de regard de référence correspond à une direction d'observation de l'individu d'une cible éloignée (vision de loin) quand l'individu est dans une posture naturelle.

Dans le mode de réalisation préféré, la direction de regard de référence est une direction de regard moyenne de l'individu 1 au cours du test visuel.

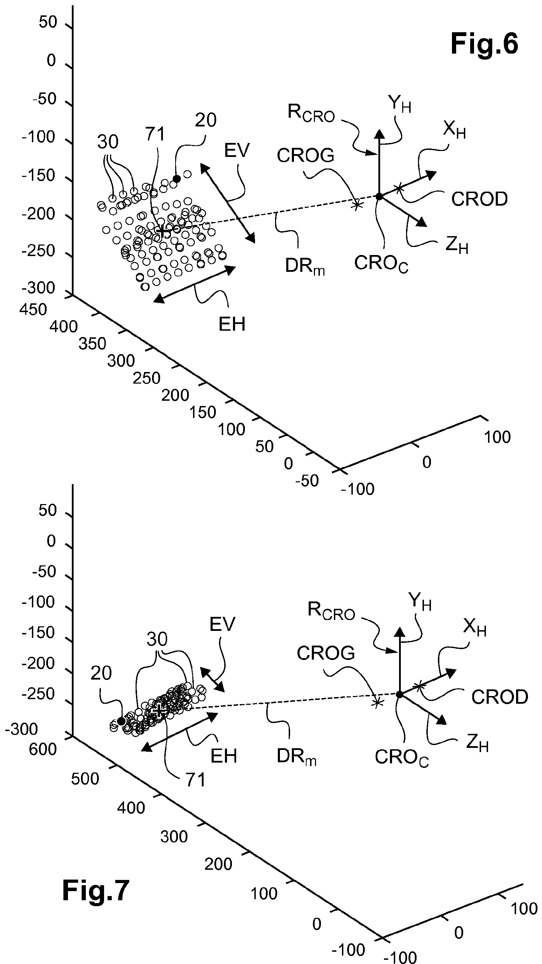

Comme représentée sur les figures 6 et 7, cette direction de regard moyenne, ci-après référencée DRₘ, est choisie de préférence comme étant la droite reliant le CRO cyclope CRO_{c} au barycentre 71 des positions cibles 30.

En variante, la direction de regard moyenne peut être définie à partir du centre de rotation droit CROD ou du centre de rotation gauche CROG.

En variante encore, la direction de regard moyenne est choisie ici comme étant la droite reliant un centre de rotation de l'œil gauche ou droit de l'individu, ou un barycentre desdits centres de rotation, à une position cible dans le repère lié à la tête de l'individu.

Compte tenu du fait non seulement que la position et l'orientation de la tête 4 de l'individu 1 change au cours du protocole de test visuel par rapport au repère R_{SCR} lié à l'appareil de capture d'image 13 mais aussi que l'individu 1 modifie la position et l'orientation de la tablette 10 au cours du test visuel, on comprend que les positions cibles 30 de la cible 20 dans le repère R_{CRO} lié à la tête 4 de l'individu 1 renseignent sur le comportement visuel de l'individu 1, en particulier sur sa propension à bouger ses yeux 3 lors de la lecture d'un texte.

En effet, si l'individu 1 suit le protocole de suivi visuel en modifiant beaucoup sa direction de regard DR, alors les positions cibles 30 de la cible 20 dans le repère R_{CRO} lié à la tête 4 de l'individu 1 sont agencées de manière relativement semblable aux positions cibles 30 de la cible 20 dans le repère R_{SCR} lié à la caméra frontale 13. Ceci est le cas de la figure 6.

À l'inverse, si l'individu 1 suit le protocole de suivi visuel en conservant une direction de regard DR *quasi* fixe, alors les positions cibles 30 de la cible 20 dans le repère R_{CRO} lié à la tête 4 de l'individu 1 sont regroupées. Ceci est le cas de la figure 7.

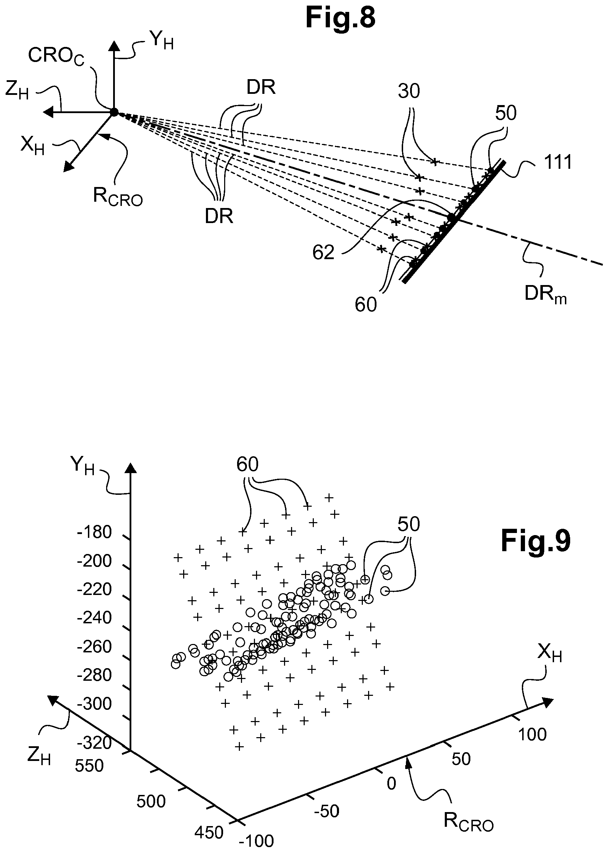

La méthode de détermination de l'invention comporte par ailleurs une étape de positionnement, par rapport à la direction de regard de référence DRₘ, de positions cibles mesurées 50 (voir figure 8) qui sont déterminées à partir des directions de regard DR de l'individu 1 mesurées au cours du test visuel lorsque l'individu 1 suit les positions cibles 30 de la cible 20 disposées sur l'écran 11 de la tablette 10.

De préférence, lors de cette étape de positionnement, on détermine également une surface d'affichage fictive 111 orientée, par rapport à la direction de regard de référence DRₘ, selon une orientation moyenne de l'écran 11 lors du test visuel.

L'orientation moyenne peut par exemple tenir compte des angles d'inclinaison et/ou de tangage moyens avec lesquels l'individu 1 tient la tablette 10 entre ses mains 2 au cours du test visuel.

Comme représenté sur la figure 8, on détermine également lors de l'étape de positionnement, les positions cibles mesurées 50 (symboles « • » sur la figure 8) comme les intersections des directions de regard DR de l'individu 1 au cours du test visuel et de la surface d'affichage fictive 111.

En d'autres termes, les positions cibles mesurées 50 correspondent aux projections des positions cibles 30, le long des directions de regard DR associées à ces positions cibles 30.

Dans un mode de réalisation préféré, la méthode de détermination comporte une étape additionnelle de positionnement.

Lors de cette étape additionnelle de positionnement, on positionne, par rapport à la direction de regard de référence, ici la direction de regard moyenne DRₘ, des positions cibles théoriques 60 (symboles « + » sur la figure 8) dont les dispositions relatives les unes par rapport aux autres sont identiques aux dispositions relatives des positions cibles 30 sur la surface d'affichage 11 (écran) de la tablette 10.

De préférence, on positionne ces positions cibles théoriques 60 de sorte que leur barycentre 62 se trouve sur la direction de regard de référence DRₘ.

Ainsi, à l'issue des étapes de positionnement décrites ci-dessus, on a déterminé, sur la surface d'affichage fictive 111, les coordonnées des positions cibles mesurées 50, et les coordonnées des positions cibles théoriques 60, dans le repère R_{CRO} lié à la tête 4 de l'individu 1. Ceci est illustré sur la figure 9 des dessins.

Des paramètres de comportement visuel de l'individu 1 lors du protocole de suivi visuel peuvent être déduits des positions cibles mesurées 50 et des positions cibles théoriques 60.

En effet, on peut déjà déterminer un premier paramètre de comportement visuel correspondant à la position (coordonnées) du barycentre (ci-après référencé NVB pour *« Near-Vision Behaviour »* en anglais) des positions cibles 30 dans le repère R_{CRO} lié à la tête 4 de l'individu 1. Ce barycentre NVB renseigne notamment sur la direction de regard moyenne DRₘ de l'individu 1 (cf. ci-dessus) lors du test visuel.

Par ailleurs, comme expliqué ci-dessus en référence aux figures 6 et 7, on comprend que la distribution (position et étalement) des positions cibles mesurées 50 par rapport aux points cibles théoriques 60, dont la distribution sur la surface d'affichage fictive 111 est fixée par celle des positions cibles 30 sur l'écran 11, renseigne sur la tendance de l'individu 1 à bouger la tête 4 et/ou les yeux 3 lors d'une tâche de lecture.

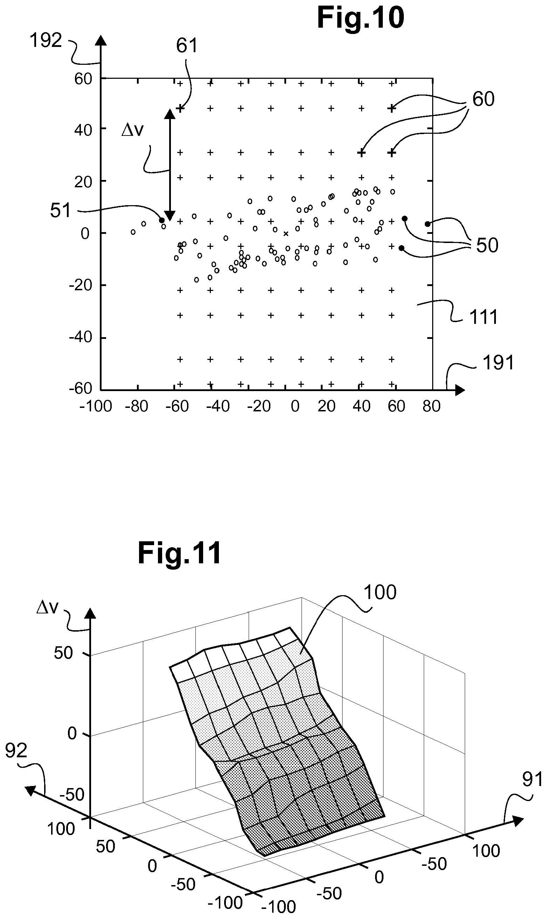

Ainsi, dans un autre mode de réalisation décrit en référence aux figures 10 à 13, l'étape de déduction de la méthode de détermination comporte de préférence une comparaison des positions cibles théoriques 60 et des positions cibles mesurées 50 dans le repère R_{CRO} lié à la tête 4 de l'individu 1. Cette comparaison permet de déduire un ou plusieurs paramètres de comportement visuel recherchés, en particulier des paramètres de comportement visuel de l'individu 1 qui sont représentatifs de l'étalement vertical EV et de l'étalement horizontal EH (voir figure 6) des positions cibles 30 dans le repère R_{CRO} lié à la tête 4 de l'individu 1. L'étalement vertical EV, respectivement l'étalement horizontal EH, est en effet représentatif de la propension de l'individu 1 à bouger ses yeux de haut en bas (ou de bas en haut), respectivement de gauche à droite (ou de droite à gauche), lors de la tâche visuelle.

Dans un mode de réalisation préféré, cette comparaison peut comprendre la détermination d'écarts entre les positions cibles théoriques 60 et les positions cibles mesurées 50 selon une direction privilégiée de la surface fictive 111. Ceci est illustré sur les figures 10 à 13.

En particulier, on a représenté sur la figure 10 la surface d'affichage fictive 111 munie d'axes 191, 192 orientés et normés de manière identiques aux axes 91, 92 de l'écran 11 (surface d'affichage réelle), les positions cibles mesurées 50 (symboles « • ») ainsi que les positions cibles théoriques 60 (symboles « + ») correspondantes.

On peut choisir par exemple comme direction privilégiée de la surface fictive 111, la direction verticale de l'axe 192.

Alors, pour chaque couple formé d'une position cible mesurée 51 et d'une position cible théorique 61 correspondant à la même position cible 30 du protocole de suivi visuel, on calcule un écart vertical, noté ici Δv, correspondant à la distance, selon la direction verticale, entre la position cible mesurée 51 et la position cible théorique 61 dudit couple.

On peut alors représenter (voir figure 11), pour chaque position cible 30 correspondant à un couple, l'ensemble des écarts verticaux Δv dans le repère R_{SCR} lié à la surface d'affichage 11 réelle. Cet ensemble est représenté par la surface 100 de la figure 11.

On pourrait également choisir une direction horizontale privilégiée (selon l'axe 191 de la figure 10) et calculer des écarts horizontaux plutôt que verticaux.

De manière avantageuse, on réalise un traitement statistique des écarts calculés pour déterminer le paramètre de comportement visuel.

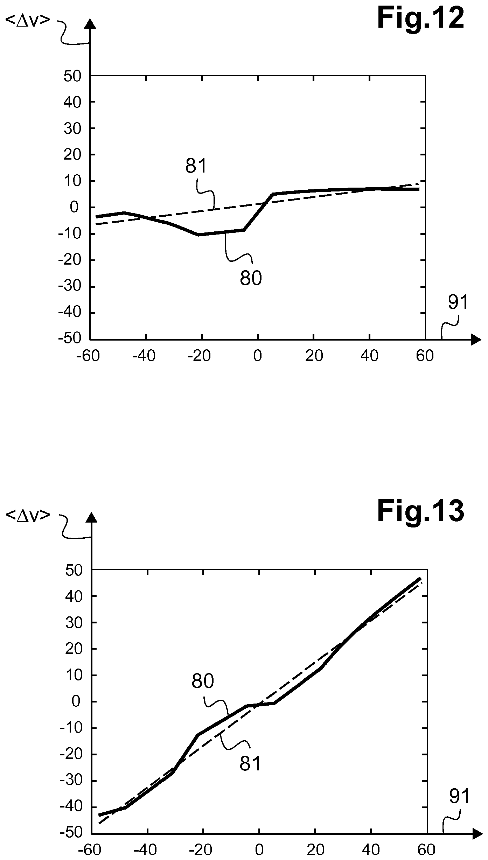

Ce traitement statistique peut par exemple comprendre les opérations suivantes :
- réaliser une moyenne < Δv > par ligne d'affichage, des écarts verticaux Δv. On obtient alors des courbes mesurées 80 telles que représentées sur les figures 12 et 13 où la moyenne < Δv > est fonction de l'index de colonne ;
- effectuer une régression linéaire afin de trouver une droite approchante 81 qui minimise l'écart avec les courbes mesurées 80.

Le coefficient directeur de cette droite approchante 81 fournit un paramètre de comportement visuel de l'individu 1 lors du protocole de test visuel.

Ce coefficient directeur est en particulier déterminé pour se situer entre 0 et 1. Pour cela, on détermine une valeur seuil minimale et une valeur seuil maximale permettant, pour des facilités d'utilisation, de normer le coefficient. Ainsi on recalcule le rapport (coefficient directeur - valeur minimale / valeur maximale - valeur minimale).

Les valeurs maximale et minimale peuvent être obtenues à partir d'une distribution de coefficients directeurs préenregistrés ou obtenues à partir de plusieurs individus.

En effet, lorsque ce coefficient directeur est faible (cas de la figure 12 avec un coefficient de 0,17), cela signifie que la moyenne des écarts entre les positions cibles mesurées 50 et les positions cibles théoriques 60 est faible. Ceci correspond au comportement visuel d'un individu 1 bougeant beaucoup les yeux 3 lors du test visuel.

À l'inverse, lorsque ce coefficient directeur est élevé (cas de la figure 13 avec un coefficient de 0,83), cela signifie que la moyenne des écarts entre les positions cibles mesurées 50 et les positions cibles théoriques 60 est élevée. Ceci correspond au comportement visuel d'un individu 1 bougeant peu les yeux 3 lors du test visuel.

Les paramètres de comportement visuel déterminés par la méthode décrite, en particulier la donnée du barycentre des positions cibles dans le repère R_{CRO} lié à la tête 4 de l'individu 1 et les paramètres d'étalement vertical EV et horizontal EH (voir figure 6) liés au calcul des coefficients directeurs (selon les lignes ou les colonnes) détaillé ci-dessus, peuvent être utilisés pour la mise en œuvre d'une méthode de conception optique d'une lentille ophtalmique de correction visuelle de l'individu 1.

De préférence, on réalise, lors de l'étape de déduction, les sous-étapes suivantes :
- on détermine une position relative d'une surface ou d'une ligne liée soit à une monture choisie par l'individu et équipée de ladite lentille ophtalmique, soit à ladite lentille ophtalmique dans le repère lié à la tête de l'individu,
- on détermine, pour chaque direction de regard de l'individu correspondant à une position cible, l'intersection entre cette direction du regard et ladite surface ou ladite ligne, de manière à établir une cartographie de ces points d'intersection avec ladite surface ou ladite ligne,
- on déduit de cette cartographie un paramètre de conception optique recherché.

Lors de la première sous-étape, on positionne de préférence le plan de la lentille ophtalmique ou le plan moyen de la monture suivant les six degrés de liberté dans le repère R_{CRO} lié à la tête 4 de l'individu 1. Il peut par exemple s'agir d'un positionnement par essayage virtuel de la monture sur un modèle de la tête 4 de l'individu 1.

## Revendications

1. Méthode de détermination d'au moins un paramètre (NVB, EV, EH) de comportement visuel d'un individu (1) comprenant les étapes suivantes :
- une étape de sollicitation de l'individu (1) pour qu'il effectue un test visuel au cours duquel il observe au moins une position cible (30),
- une étape de mesure d'au moins une donnée représentative d'au moins une direction de regard (DR) de l'individu (1) au cours dudit test visuel,
- une étape de détermination d'une direction de regard de référence (DRₘ), en fonction de ladite au moins une donnée représentative mesurée,
la méthode **caractérisée par** :
- une étape de positionnement, par rapport à ladite direction de regard de référence (DRₘ), d'au moins une position cible mesurée (50) qui est déterminée, dans un repère (R_{CRO}) lié à la tête (4) de l'individu (1), en fonction de ladite donnée représentative de ladite direction de regard (DR) de l'individu (1) mesurée au cours du test visuel.

2. Méthode selon la revendication 1, selon laquelle on réalise, après ladite étape de positionnement, une étape de déduction, en fonction de ladite au moins une position cible mesurée (50) et/ou de la direction de regard de référence (DRₘ), dudit paramètre (NVB, EV, EH) de comportement visuel de l'individu (1).

3. Méthode selon la revendication 1 ou 2, selon laquelle ladite direction de regard de référence (DRₘ) est une direction de regard moyenne de l'individu (1) au cours du test visuel.

4. Méthode selon la revendication 1 ou 2, selon laquelle ladite direction de regard de référence (DRₘ) correspond à une direction d'observation d'une cible éloignée quand l'individu est dans une posture naturelle.

5. Méthode selon l'une des revendications 1 à 4, selon laquelle, à l'étape de sollicitation, l'individu (1) observe successivement différentes positions cibles (30).

6. Méthode selon la revendication 5, selon laquelle :
- on détermine lesdites directions de regard (DR) de l'individu (1) au cours du test visuel dans ledit repère (R_{CRO}) lié à la tête (4) de l'individu (1),
- on détermine les coordonnées desdites positions cibles (30) dans ledit repère (R_{CRO}) lié à la tête (4) de l'individu (1), et
- on détermine ladite direction de regard de référence (DRₘ) comme étant une droite reliant un centre de rotation de l'œil gauche (CROG) ou droit (CROD) de l'individu (1), ou un barycentre (CRO_{C}) desdits centres de rotation, à une position cible (30) dans le repère (R_{CRO}) lié à la tête (4) de l'individu (1).

7. Méthode selon la revendication 5, selon laquelle :
- on détermine lesdites directions de regard (DR) de l'individu (1) au cours du test visuel dans un repère (R_{CRO}) lié à la tête (4) de l'individu (1),
- on détermine les coordonnées desdites positions cibles (30) dans ledit repère (R_{CRO}) lié à la tête (4) de l'individu (1),
- on détermine, à partir desdites coordonnées, un barycentre (NVB) desdites positions cibles (30) dans le repère (R_{CRO}) lié à la tête (4) de l'individu (1), et
- on détermine ladite direction de regard de référence (DRₘ) comme une droite reliant un centre de rotation d'un œil gauche (CROG) ou droit (CROD) de l'individu (1), ou un barycentre (CRO_{C}) desdits centres de rotation, audit barycentre (NVB) des positions cibles (30) dans le repère (R_{CRO}) lié à la tête (4) de l'individu (1).

8. Méthode selon la revendication 6 ou 7, selon laquelle ledit repère (R_{CRO}) lié à la tête (4) de l'individu (1) a pour origine l'un des centres de rotation (CROD, CROG) de l'œil droit ou de l'œil gauche de l'individu (1) ou un barycentre (CRO_{C}) desdits centres de rotation.

9. Méthode selon la revendication 8, selon laquelle ledit repère (R_{CRO}) lié à la tête (4) de l'individu (1) comprend :
- un premier axe (Z_{H}) qui est parallèle à une direction de regard primaire de l'individu (1) ;
- un deuxième axe (X_{H}) qui est horizontal et perpendiculaire audit premier axe (Z_{H}), et
- un troisième axe (Y_{H}) qui est perpendiculaire auxdits premier (X_{H}) et deuxième axe (Z_{H}).

10. Méthode selon l'une des revendications 5 à 9, comportant une étape additionnelle de positionnement, par rapport à ladite direction de regard de référence (DRₘ), de positions cibles théoriques (60) dont les dispositions relatives les unes par rapport aux autres sont identiques aux dispositions relatives desdites positions cibles (30).

11. Méthode selon la revendication 10, selon laquelle, lors de ladite étape additionnelle de positionnement, on positionne lesdites positions cibles théoriques (60) de sorte que le barycentre (62) desdites positions cibles théoriques (60) se trouve sur la direction de regard de référence (DRₘ).

12. Méthode selon la revendication 10 ou 11, selon laquelle, lors du test visuel, les positions cibles (30) sont disposées sur une surface d'affichage (11), et, lors de l'étape de positionnement, on détermine une surface d'affichage fictive (111) orientée, par rapport à ladite direction de regard de référence (DRₘ), selon une orientation moyenne de ladite surface d'affichage (11) lors du test visuel et on détermine lesdites positions cibles mesurées (50) comme les intersections desdites directions de regard (DR) de l'individu (1) au cours du test visuel et de ladite surface d'affichage fictive (111).

13. Méthode selon l'une des revendications 10 à 12 en dépendance de la revendication 2, selon laquelle lors de l'étape de déduction, on compare lesdites positions cibles théoriques (60) et lesdites positions cibles mesurées (50) et on en déduit ledit paramètre (NVB, EV, EH) de comportement visuel de l'individu (1).

14. Méthode selon la revendication 12 en dépendance de la revendication 2 ou selon la revendication 13, selon laquelle lors de l'étape de déduction, on détermine des écarts (Δv) entre lesdites positions cibles théoriques (60) et lesdites positions cibles mesurées (50) selon une direction privilégiée de ladite surface fictive (111) et on en déduit ledit paramètre (NVB, EV, EH) de comportement visuel de l'individu (1).

15. Méthode selon l'une des revendications 13 et 14, selon laquelle, à l'étape de déduction, ledit paramètre (NVB, EV, EH) de comportement visuel est déterminé en fonction d'un traitement statistique des écarts (Δv) entre lesdites positions cibles théoriques (60) et lesdites positions cibles mesurées (50).

16. Méthode selon l'une des revendications 5 à 15, selon laquelle à l'étape de mesure :
- on capture, au moyen d'un appareil de capture d'image (13), au moins une image d'une partie de la tête (4) de l'individu (1) observant chaque position cible (30),
- on mémorise ladite au moins une image en association avec les coordonnées, exprimées dans un repère (R_{SCR}) lié à l'appareil de capture d'image (13), de la position cible (30) observée par l'individu (1),
- on détermine, à partir de ladite au moins une image capturée et des coordonnées associées de la position cible (30) observée, les coordonnées d'un repère (R_{CRO}) lié à la tête (4) de l'individu (1) dans le repère (R_{SCR}) lié audit appareil de capture d'image (13) ou les coordonnées des directions de regard (DR) de l'individu (1) dans le repère (R_{CRO}) lié à la tête (4) de l'individu (1).

17. Méthode selon l'une des revendications 1 à 16, selon laquelle chaque direction de regard (DR) de l'individu (1) est définie comme la droite passant, d'une part, par l'un des centres de rotation (CROD, CROG) de l'œil droit ou de l'œil gauche de l'individu (1) ou le barycentre (CRO_{C}) desdits centres de rotation et, d'autre part, par la position cible (30) observée par l'individu (1) au moment de la mesure.

18. Méthode selon l'une des revendications 1 à 17, selon laquelle le test visuel effectué par l'individu (1) comporte une tâche visuelle en vision de près destinée à tester le comportement visuel de l'individu (1) en situation de lecture d'un texte.

19. Méthode selon la revendication 18 en dépendance de la revendication 5, selon laquelle lesdites positions cibles (30) sont alignées selon au moins deux lignes (L1, L2, L3, L4, L5) ou deux colonnes sensiblement parallèles et les positions cibles (30) observées successivement, au cours du temps, par l'individu (1) définissent un protocole de suivi visuel destiné à reproduire le déplacement du regard de l'individu (1) pendant la lecture.

20. Méthode de conception optique d'une lentille ophtalmique de correction visuelle d'un individu, utilisant le paramètre (NVB, EV, EH) de comportement visuel déterminé selon la méthode de détermination de l'une des revendications 1 à 19.

21. Méthode de conception optique selon la revendication 20, selon laquelle, ladite lentille ophtalmique de correction visuelle étant destinée à équiper une monture choisie par l'individu (1), on réalise, lors de l'étape de déduction de la méthode de détermination du paramètre (NVB, EV, EH) de comportement visuel, les sous-étapes suivantes :
- on détermine une position relative d'une surface ou d'une ligne liée à ladite monture ou à ladite lentille ophtalmique dans le repère (R_{CRO}) lié à la tête (4) de l'individu (1),
- on détermine, pour chaque direction de regard (DR) de l'individu (1) correspondant à une position cible (30), l'intersection entre cette direction du regard et ladite surface ou ladite ligne, de manière à établir une cartographie de ces points d'intersection avec ladite surface ou ladite ligne,
- on déduit de cette cartographie un paramètre de conception optique recherché.

22. Dispositif de test (10) adapté pour mettre en œuvre de la méthode selon l'une des revendications 1 à 19, comportant :
- un afficheur (11) actif adapté à afficher au moins une cible (20) visuellement prépondérante en une pluralité de positions cibles (30) alignées selon au moins deux lignes (L1, L2, L3, L4, L5) ou colonnes sensiblement parallèles,
- une unité de commande de l'afficheur (11), programmée pour que les positions cibles (30) définissent, au cours du temps, un protocole de suivi visuel de manière à reproduire le déplacement du regard de l'individu (1) pendant la lecture, et
- un appareil de capture d'image (13) piloté par ladite unité de commande de manière synchrone avec ledit afficheur (11) pour déclencher des captures d'images de la tête (4) de l'individu (1) observant ladite cible (20) affichée par l'afficheur (11), chaque image capturée correspondant à une position cible (30) prédéterminée.

## Patentansprüche

1. Verfahren zur Bestimmung mindestens eines visuellen Verhaltensparameters (NVB, EV, EH) einer Person (1), das die folgenden Schritte enthält:
- einen Schritt der Aufforderung der Person (1), einen Sehtest auszuführen, während dessen sie mindestens eine Zielposition (30) betrachtet,
- einen Schritt des Messens mindestens eines Datenwerts, der für mindestens eine Blickrichtung (DR) der Person (1) während des Sehtests repräsentativ ist,
- einen Schritt der Bestimmung einer Bezugsblickrichtung (DRₘ) abhängig von dem mindestens einen gemessenen repräsentativen Datenwert,
wobei das Verfahren **gekennzeichnet ist durch**:
- einen Schritt der Positionierung, bezüglich der Bezugsblickrichtung (DRₘ), mindestens einer gemessenen Zielposition (50), die in einem mit dem Kopf (4) der Person (1) verbundenen Bezugssystem (R_{CRO}) abhängig vom gemessenen, für die Blickrichtung (DR) der Person (1) während des Sehtests repräsentativen Datenwert bestimmt wird.

2. Verfahren nach Anspruch 1, wobei nach dem Positionierungsschritt ein Schritt der Ableitung, abhängig von der mindestens einen gemessenen Zielposition (50) und/oder der Bezugsblickrichtung (DRₘ), des visuellen Verhaltensparameters (NVB, EV, EH) der Person (1) durchgeführt wird.

3. Verfahren nach Anspruch 1 oder 2, wobei die Bezugsblickrichtung (DRₘ) eine mittlere Blickrichtung der Person (1) während des Sehtests ist.

4. Verfahren nach Anspruch 1 oder 2, wobei die Bezugsblickrichtung (DRₘ) einer Betrachtungsrichtung eines entfernten Ziels entspricht, wenn die Person in einer natürlichen Haltung ist.

5. Verfahren nach einem der Ansprüche 1 bis 4, wobei die Person (1) im Schritt der Aufforderung nacheinander verschiedene Zielpositionen (30) betrachtet.

6. Verfahren nach Anspruch 5, wobei:
- die Blickrichtungen (DR) der Person (1) während des Sehtests im mit dem Kopf (4) der Person (1) verbundenen Bezugssystem (R_{CRO}) bestimmt werden,
- die Koordinaten der Zielpositionen (30) im mit dem Kopf (4) der Person (1) verbundenen Bezugssystem (R_{CRO}) bestimmt werden, und
- die Bezugsblickrichtung (DRₘ) als eine Gerade bestimmt wird, die einen Drehpunkt des linken (CROG) oder rechten Auges (CROD) der Person (1) oder ein Baryzentrum (CRO_{C}) der Drehpunkte mit einer Zielposition (30) in dem mit dem Kopf (4) der Person (1) verbundenen Bezugssystem (R_{CRO}) verbindet.

7. Verfahren nach Anspruch 5, wobei:
- die Blickrichtungen (DR) der Person (1) während des Sehtests in einem mit dem Kopf (4) der Person (1) verbundenen Bezugssystem (R_{CRO}) bestimmt werden,
- die Koordinaten der Zielpositionen (30) im mit dem Kopf (4) der Person (1) verbundenen Bezugssystem (R_{CRO}) bestimmt werden,
- ausgehend von den Koordinaten ein Baryzentrum (NVB) der Zielpositionen (30) in dem mit dem Kopf (4) der Person (1) verbundenen Bezugssystem (R_{CRO}) bestimmt wird, und
- die Bezugsblickrichtung (DRₘ) als eine Gerade bestimmt wird, die einen Drehpunkt eines linken (CROG) oder rechten Auges (CROD) der Person (1) oder ein Baryzentrum (CRO_{C}) der Drehpunkte mit dem Baryzentrum (NVB) der Zielpositionen (30) in dem mit dem Kopf (4) der Person (1) verbundenen Bezugssystem (R_{CRO}) verbindet.

8. Verfahren nach Anspruch 6 oder 7, wobei das mit dem Kopf (4) der Person (1) verbundene Bezugssystem (R_{CRO}) einen der Drehpunkte (CROD, CROG) des rechten oder linken Auges der Person (1) oder ein Baryzentrum (CRO_{C}) der Drehpunkte als Ursprung hat.

9. Verfahren nach Anspruch 8, wobei das mit dem Kopf (4) der Person (1) verbundene Bezugssystem (R_{CRO}) enthält:
- eine erste Achse (Z_{H}), die zu einer primären Blickrichtung der Person (1) parallel ist;
- eine zweite Achse (X_{H}), die waagrecht und lotrecht zur ersten Achse (Z_{H}) ist, und
- eine dritte Achse (Y_{H}), die lotrecht zu der ersten (X_{H}) und zweiten Achse (Z_{H}) ist.

10. Verfahren nach einem der Ansprüche 5 bis 9, das einen zusätzlichen Schritt der Positionierung, bezüglich der Bezugsblickrichtung (DRₘ), von theoretischen Zielpositionen (60) aufweist, deren zueinander relative Anordnungen gleich den relativen Anordnungen der Zielpositionen (30) sind.

11. Verfahren nach Anspruch 10, wobei die theoretischen Zielpositionen (60) im zusätzlichen Positionierschritt so positioniert werden, dass das Baryzentrum (62) der theoretischen Zielpositionen (60) sich in der Bezugsblickrichtung (DRₘ) befindet.

12. Verfahren nach Anspruch 10 oder 11, wobei die Zielpositionen (30) beim Sehtest auf einer Anzeigefläche (11) angeordnet sind, und im Positionierungsschritt eine fiktive Anzeigefläche (111) bestimmt wird, die bezüglich der Bezugsblickrichtung (DRₘ) gemäß einer mittleren Ausrichtung der Anzeigefläche (11) beim Sehtest ausgerichtet ist, und die gemessenen Zielpositionen (50) als die Schnittstellen der Blickrichtungen (DR) der Person (1) während des Sehtests und der fiktiven Anzeigefläche (111) bestimmt werden.

13. Verfahren nach einem der Ansprüche 10 bis 12 abhängig von Anspruch 2, wobei im Ableitungsschritt die theoretischen Zielpositionen (60) und die gemessenen Zielpositionen (50) verglichen werden, und der visuelle Verhaltensparameter (NVB, EV, EH) der Person (1) daraus abgeleitet wird.

14. Verfahren nach Anspruch 12 abhängig von Anspruch 2 oder nach Anspruch 13, wobei im Ableitungsschritt Abweichungen (Δv) zwischen den theoretischen Zielpositionen (60) und den gemessenen Zielpositionen (50) gemäß einer bevorzugten Richtung der fiktiven Fläche (111) bestimmt werden, und der visuelle Verhaltensparameter (NVB, EV, EH) der Person (1) daraus abgeleitet wird.

15. Verfahren nach einem der Ansprüche 13 und 14, wobei im Ableitungsschritt der visuelle Verhaltensparameter (NVB, EV, EH) abhängig von einer statistischen Verarbeitung der Abweichungen (Δv) zwischen den theoretischen Zielpositionen (60) und den gemessenen Zielpositionen (50) bestimmt wird.

16. Verfahren nach einem der Ansprüche 5 bis 15, wobei im Messschritt:
- mittels eines Bilderfassungsgeräts (13) mindestens ein Bild eines Bereichs des Kopfs (4) der Person (1) erfasst wird, die jede Zielposition (30) betrachtet,
- das mindestens eine Bild in Verbindung mit den in einem mit dem Bilderfassungsgerät (13) verbundenen Bezugssystem (R_{SCR}) ausgedrückten Koordinaten der von der Person (1) betrachteten Zielposition (30) gespeichert wird,
- ausgehend von dem mindestens einen erfassten Bild und den zugeordneten Koordinaten der betrachteten Zielposition (30) die Koordinaten eines mit dem Kopf (4) der Person (1) verbundenen Bezugssystems (R_{CRO}) im mit dem Bilderfassungsgerät (13) verbundenen Bezugssystem (R_{SCR}) oder die Koordinaten der Blickrichtungen (DR) der Person (1) im mit dem Kopf (4) der Person (1) verbundenen Bezugssystem (R_{CRO}) bestimmt werden.

17. Verfahren nach einem der Ansprüche 1 bis 16, wobei jede Blickrichtung (DR) der Person (1) als die Gerade definiert wird, die einerseits durch einen der Drehpunkte (CROD, CROG) des rechten Auges oder des linken Auges der Person (1) oder das Baryzentrum (CRO_{C}) der Drehpunkte und andererseits durch die Zielposition (30) verläuft, die von der Person (1) zum Zeitpunkt der Messung betrachtet wird.

18. Verfahren nach einem der Ansprüche 1 bis 17, wobei der von der Person (1) ausgeführte Sehtest eine visuelle Aufgabe in Nahsicht aufweist, die dazu bestimmt ist, das visuelle Verhalten der Person (1) in der Situation des Lesens eines Textes zu testen.

19. Verfahren nach Anspruch 18 abhängig von Anspruch 5, wobei die Zielpositionen (30) gemäß mindestens zwei im Wesentlichen parallelen Zeilen (L1, L2, L3, L4, L5) oder zwei Spalten ausgerichtet sind, und die nacheinander im Zeitverlauf von der Person (1) betrachteten Zielpositionen (30) ein visuelles Verfolgungsprotokoll definieren, das dazu bestimmt ist, die Verschiebung des Blicks der Person (1) während des Lesens zu reproduzieren.

20. Optisches Gestaltungsverfahren einer ophthalmischen Sehkorrekturlinse einer Person, das den visuellen Verhaltensparameter (NVB, EV, EH) verwendet, der gemäß dem Bestimmungsverfahren eines der Ansprüche 1 bis 19 bestimmt wird.

21. Optisches Gestaltungsverfahren nach Anspruch 20, wobei, da die ophthalmische Sehkorrekturlinse dazu bestimmt ist, ein von der Person (1) ausgewähltes Gestell zu bestücken, beim Ableitungsschritt des Verfahrens zur Bestimmung des visuellen Verhaltensparameters (NVB, EV, EH) die folgenden Teilschritte durchgeführt werden:
- es wird eine relative Position einer mit dem Gestell oder mit der ophthalmischen Linse verbundenen Fläche oder Linie in dem mit dem Kopf (4) der Person (1) verbundenen Bezugssystem (R_{CRO}) bestimmt,
- für jede Blickrichtung (DR) der Person (1) entsprechend einer Zielposition (30) wird die Schnittstelle zwischen dieser Richtung des Blicks und der Fläche oder der Linie bestimmt, um eine Kartographie dieser Schnittpunkte mit der Fläche oder der Linie zu erstellen,
- von dieser Kartographie wird ein gesuchter optischer Gestaltungsparameter abgeleitet.

22. Testvorrichtung, die geeignet ist, das Verfahren nach einem der Ansprüche 1 bis 19 durchzuführen, die aufweist:
- ein aktives Anzeigegerät (11), das geeignet ist, mindestens ein visuell herausragendes Ziel (20) in einer Vielzahl von Zielpositionen (30) anzuzeigen, die gemäß mindestens zwei im Wesentlichen parallelen Zeilen (L1, L2, L3, L4, L5) oder Spalten ausgerichtet sind,
- eine Steuereinheit des Anzeigegeräts (11), die programmiert ist, damit die Zielpositionen (30) im Zeitverlauf ein visuelles Verfolgungsprotokoll definieren, um die Verschiebung des Blicks der Person (1) während des Lesens zu reproduzieren, und
- ein von der Steuereinheit synchron mit dem Anzeigegerät (11) gesteuertes Bilderfassungsgerät (13), um Erfassungen von Bildern des Kopfes (4) der Person (1) auszulösen, die das vom Anzeigegerät (11) angezeigte Ziel (20) betrachtet, wobei jedes erfasste Bild einer vorbestimmten Zielposition (30) entspricht.

## Claims

1. Method for determining at least one parameter (NVB, EV, EH) of the visual behaviour of an individual (1) comprising the following steps:
- a step of requesting the individual (1) so that he performs a visual test during which he observes at least one target position (30),
- a step of measuring at least one datum representative of at least one direction of gaze (DR) of the individual (1) during the said visual test,
- a step of determining a reference direction of gaze (DRₘ), as a function of the said at least one measured representative datum,
the method being **characterized by**:
- a step of positioning, with respect to the said reference direction of gaze (DRₘ), at least one measured target position (50) which is determined, in a reference frame (R_{CRO}) related to the head (4) of the individual (1), as a function of the said datum representative of the said direction of gaze (DR) of the individual (1) measured during the visual test.

2. Method according to Claim 1, wherein, after the said positioning step, a step is carried out of deducing, depending on the said at least one measured target position (50) and/or on the reference direction of gaze (DRₘ), the said parameter (NVB, EV, EH) of the visual behaviour of the individual (1).

3. Method according to Claim 1 or 2, wherein the said reference direction of gaze (DRₘ) is a mean direction of gaze of the individual (1) during the visual test.

4. Method according to Claim 1 or 2, wherein the said reference direction of gaze (DRₘ) corresponds to a direction of observation of a distant target when the individual is in a natural posture.

5. Method according to one of Claims 1 to 4, according to which, in the requesting step, the individual (1) successively observes various target positions (30).

6. Method according to Claim 5, according to which:
- the said directions of gaze (DR) of the individual (1) are determined during the visual test in the said reference frame (R_{CRO}) related to the head (4) of the individual (1),
- the coordinates of the said target positions (30) are determined in the said reference frame (R_{CRO}) related to the head (4) of the individual (1), and
- the said reference direction of gaze (DRₘ) is determined as being a straight line linking a center of rotation of the left eye (CROG) or right eye (CROD) of the individual (1), or a barycenter (CRO_{C}) of the said centers of rotation, to a target position (30) in the reference frame (R_{CRO}) related to the head (4) of the individual (1).

7. Method according to Claim 5, according to which:
- the said directions of gaze (DR) of the individual (1) are determined during the visual test in a reference frame (R_{CRO}) related to the head (4) of the individual (1),
- the coordinates of the said target positions (30) are determined in the said reference frame (R_{CRO}) related to the head (4) of the individual (1),
- a barycenter (NVB) of the said target positions (30) in the reference frame (R_{CRO}) related to the head (4) of the individual (1) is determined on the basis of the said coordinates, and
- the said reference direction of gaze (DRₘ) is determined as a straight line linking a center of rotation of a left eye (CROG) or right eye (CROD) of the individual (1), or a barycenter (CRO_{C}) of the said centers of rotation, to the said barycenter (NVB) of the target positions (30) in the reference frame (R_{CRO}) related to the head (4) of the individual (1).

8. Method according to Claim 6 or 7, wherein the origin of the said reference frame (R_{CRO}) related to the head (4) of the individual (1) is one of the centers of rotation (CROD, CROG) of the right eye or of the left eye of the individual (1) or a barycenter (CRO_{C}) of the said centers of rotation.

9. Method according to Claim 8, wherein the said reference frame (R_{CRO}) related to the head (4) of the individual (1) comprises:
- a first axis (Z_{H}) which is parallel to a primary direction of gaze of the individual (1),
- a second axis (X_{H}) which is horizontal and perpendicular to the said first axis (Z_{H}), and
- a third axis (Y_{H}) which is perpendicular to the said first axis (X_{H}) and to the said second axis (Z_{H}).

10. Method according to one of Claims 5 to 9, comprising an additional step of positioning, with respect to the said reference direction of gaze (DRₘ), theoretical target positions (60) whose relative dispositions with respect to one another are identical to the relative dispositions of the said target positions (30).

11. Method according to Claim 10, wherein, during the said additional positioning step, the said theoretical target positions (60) are positioned so that the barycenter (62) of the said theoretical target positions (60) lies on the reference direction of gaze (DRₘ).

12. Method according to Claim 10 or 11, wherein, during the visual test, the target positions (30) are disposed on a display surface (11), and, during the positioning step, a dummy display surface (111) oriented, with respect to the said reference direction of gaze (DRₘ), according to a mean orientation of the said display surface (11) during the visual test is determined and the said measured target positions (50) are determined as the intersections of the said directions of gaze (DR) of the individual (1) during the visual test and of the said dummy display surface (111) .

13. Method according to one of Claims 10 to 12 when dependent on Claim 2, wherein, during the deducing step, the said theoretical target positions (60) and the said measured target positions (50) are compared and the said parameter (NVB, EV, EH) of the visual behaviour of the individual (1) is deduced therefrom.

14. Method according to Claim 12 when dependent on Claim 2 or according to Claim 13, wherein, during the deducing step, disparities (Δv) are determined between the said theoretical target positions (60) and the said measured target positions (50) according to a favoured direction of the said dummy surface (111) and the said parameter (NVB, EV, EH) of the visual behaviour of the individual (1) is deduced therefrom.

15. Method according to either of Claims 13 and 14, wherein, in the deducing step, the said visual behaviour parameter (NVB, EV, EH) is determined as a function of a statistical processing of the disparities (Δv) between the said theoretical target positions (60) and the said measured target positions (50).

16. Method according to one of Claims 5 to 15, wherein, in the measuring step:
- at least one image of a part of the head (4) of the individual (1) observing each target position (30) is captured by means of an image capture apparatus (13),
- the said at least one image is stored in association with the coordinates, expressed in a reference frame (R_{SCR}) related to the image capture apparatus (13), of the target position (30) observed by the individual (1), and
- the coordinates of a reference frame (R_{CRO}) related to the head (4) of the individual (1) in the reference frame (R_{SCR}) related to the said image capture apparatus (13) or the coordinates of the directions of gaze (DR) of the individual (1) in the reference frame (R_{CRO}) related to the head (4) of the individual (1) are determined on the basis of the said at least one captured image and of the associated coordinates of the observed target position (30).

17. Method according to one of Claims 1 to 16, wherein each direction of gaze (DR) of the individual (1) is defined as the straight line passing, on the one hand, through one of the centers of rotation (CROD, CROG) of the right eye or of the left eye of the individual (1) or the barycenter (CRO_{C}) of the said centers of rotation and, on the other hand, through the target position (30) observed by the individual (1) at the moment of the measurement.

18. Method according to one of Claims 1 to 17, wherein the visual test performed by the individual (1) includes a visual task in near vision intended to test the visual behaviour of the individual (1) in a situation in which a text is read.

19. Method according to Claim 18 when dependent on Claim 5, wherein the said target positions (30) are aligned in at least two lines (L1, L2, L3, L4, L5) or two columns that are substantially parallel and the target positions (30) observed successively, over time, by the individual (1) define a protocol for visual tracking intended to reproduce the displacement of the gaze of the individual (1) while reading.

20. Optical method for designing an ophthalmic lens for correcting the vision of an individual, using the visual behaviour parameter (NVB, EV, EH) determined according to the determining method of one of Claims 1 to 19.

21. Optical designing method according to Claim 20, wherein, the said vision-correcting ophthalmic lens being intended to be mounted in a frame chosen by the individual (1), the following substeps are carried out during the deducing step of the method for determining the visual behaviour parameter (NVB, EV, EH):
- a relative position of a surface or of a line associated with the said frame or the said ophthalmic lens is determined in the reference frame (R_{CRO}) related to the head (4) of the individual (1),
- for each direction of gaze (DR) of the individual (1) corresponding to a target position (30), the intersection between this direction of the gaze and the said surface or the said line is determined, so as to establish a map of these points of intersection with the said surface or the said line,
- a sought optical design parameter is deduced from this map.

22. Test device (10) suitable for implementing the method according to one of Claims 1 to 19, including:
- an active display (11) suitable for displaying at least one visually predominant target (20) at a plurality of target positions (30) aligned according to at least two substantially parallel lines (L1, L2, L3, L4, L5) or columns,
- a control unit for the display (11), programmed so that the target positions (30) define, over time, a protocol for visual tracking so as to reproduce the displacement of the gaze of the individual (1) while reading, and
- an image capture apparatus (13) which is driven by the said control unit in a manner synchronous with the said display (11) so as to trigger captures of images of the head (4) of the individual (1) observing the said target (20) displayed by the display (11), each captured image corresponding to a predetermined target position (30).
